# EUROPEAN PATENT SPECIFICATION

(11) **EP 3 666 279 B1**
(45) Date of publication and mention of the grant of the patent: **17.07.2024**
(21) Application number: 18211710.1
(22) Date of filing: 11.12.2018
(51) Int. Cl.: A61K 33/00, A61K 9/00, A61M 16/12

(54) **MEDICAL GAS MIXTURE**
MEDIZINISCHES GASGEMISCH
MÉLANGE DE GAZ MÉDICAL

(43) Date of publication of application: 17.06.2020
(73) Proprietor: Messer Romania Gaz SRL, 024102 Bucaresti (RO)
(72) Inventor: Bataila, Lucian, 177160 Popesti-Leordeni (IF) (RO)
(74) Representative: Münzel, Joachim R.

(56) References cited:
- US-A1- 2013 047 988
- PAUL M MACEY ET AL: "Hyperoxic Brain Effects Are Normalized by Addition of CO 2", PLOS MEDICINE, vol. 4, no. 5, 22 May 2007 (2007-05-22), pages 828 - 835, XP055589703
- NINA CA LANSDORP ET AL: "Double-blind trials in hyperbaric medicine: A narrative review on past experiences and considerations in designing sham hyperbaric treatment", CLINICAL TRIALS, vol. 15, no. 5, 4 June 2018 (2018-06-04), GB, pages 462 - 476, XP055589768, ISSN: 1740-7745, DOI: 10.1177/1740774518776952

## Description

The invention refers to a medical gas mixture comprising oxygen and carbon dioxide for use as inhalation medicinal product (inhalation drug).

Medical oxygen is one of the most widely used drugs in hospitals because a large proportion of patients are treated with oxygen at a certain time during their stay in the clinical settings. For example, very few patients who receive anaesthesia, have cardiac or respiratory disease, or are in intensive care are not given oxygen during their treatment.

Medical oxygen has been marketing for more than 100 years completely free of carbon dioxide since its introduction into therapy, being fully depleted in CO₂ for technological reasons. During the process of oxygen production in air separation units (ASU), the natural CO₂ content of the air is completely removed in order to avoid plugging of the installations with dry ice. This is completely unknown to the medical professionals, while in the gas industry field, most people who are aware of this fact do not own the necessary scientific knowledge to assess the effect of this artificial change on patients. In addition, the high purity of the oxygen produced in this way was regarded as a positive aspect. The carbon dioxide content of medical oxygen is therefore well below the carbon dioxide content of the air and below the 300 ppmv concentration permitted in both, the EU and the US Pharmacopeia.

Independently of this, clinicians have reported for decades some adverse reactions of oxygen therapy, other than the well-known oxygen toxicity. It has long been known that hyperoxia (increase in arterial oxygen partial pressure, PaO₂, usually caused by oxygen inhalation) induces hyperventilation and results in a loss of carbon dioxide content in the blood and thus, leads to hypocapnia (which herein is defined as a decrease in the arterial carbon dioxide partial pressure, PaCO₂, below the normal value). The extent of hypocapnia induced by oxygen therapy is dose dependent: the longer the duration of O₂ - administration and the higher the O₂ - partial pressure administrated, the higher is the decrease in PaCO₂ induced.

Furthermore, it is known that CO₂ is a potent vasodilator and thus, hypocapnia may lead to vasoconstriction (see: Floyd T.F., Clark J.M., Gelfand R., Detre J.A., Ratcliffe S., Guvakov D., Lambertsen C.J., Eckenhoff R.G.: Independent cerebral vasoconstrictive effects of hyperoxia and accompanying arterial hypocapnia at 1 ATA., J. Appl. Physiol. 2003; 95(6), p. 2453-2461; or, Sjöberg F, Singer M: The medical use of oxygen: a time for critical reappraisal, J. Intern. Med. 2013; 276(6), p. 505-528). The sequence hyperventilation → hypocapnia → vasoconstriction has deleterious effects for the oxygen therapy by reducing the oxygen deliveries to the tissues and consequently, by decreasing the drug efficacy.

Moreover, hypocapnia increases the pH-value and also the affinity of hemoglobin for oxygen which results in a reduction in the unloading of oxygen from arterial blood and exacerbating ischemia in that tissue (see S. Iscoe, S. and Fisher, J.A.: Hyperoxia-induced hypocapnia: An underappreciated risk. Chest 2005; 128(1): 430-433).

Regarding the hyperoxia induced vasoconstriction, its deleterious effects have been revealed by many authors over the last two decades. At issue is the principle that the purpose of oxygen therapy should be the increase of the oxygen delivery in order to reach a high oxygen level in the arterial blood. Therefore, the oxygen supply of a patient does not only depend on the arterial oxygen content, but also on the tissue perfusion. If during hyperoxia, the perfusion decreases more than the oxygen level in the arterial blood increases, the oxygen delivery in the tissue and the drug efficiacy decreases. This may be the main mechanism responsible for the worse outcomes observed in many cases where pure oxygen was given to patients in the therapy of AMI (Acute Myocardial Infarction), cardiac arrest, stroke, neonatal resuscitation, to patients in postoperative state or critically ill patients, and it could also be responsible for the increased mortality during acute episodes of ARDS (Acute Respiratory Distress Syndrome) and COPD (Chronic Obstructive Pulmonary Disease) of patients treated with pure oxygen.

It should be noted that oxygen toxicity is out of the scope of the present patent application.

Besides pure oxygen and its therapeutic applications mentioned above, various gas mixtures containing oxygen and carbon dioxide have already been used for therapeutic purposes.

Under the name "Carbogen" an inhaling gas mixture is known which consists of oxygen and an amount of 5 vol.% carbon dioxide. It is used in medical applications for artificial respiration in humans or to trigger hyperventilation, especially in the therapy of carbon monoxide poisoning, in poisoning with halogenated hydrocarbons such as dichloromethane and in cell culture for supplying tissue preparations and cultures of microorganisms with oxygen.

US 3 974 830 A describes a process and a device for a therapy against addiction which self is not addictive, including addiction to heroin, methadone and alcohol. The patient briefly inhales a heated gas mixture containing a portion of 79-80 vol.-% CO₂, the remainder being essentially oxygen, for a period typically between 30s and 120s. The gas mixture is delivered over a period of several weeks through a special mask not touching the patient's face.

EP 0 727 219 A2 describes a medical gas mixture consisting of 20 to 40 vol.-% oxygen, 2 to 10 vol.-% carbon dioxide, the remainder essentially helium. The gas mixture is used in the treatment of COPD, asthma and various other medical applications.

EP 0 868 921 A2 describes a medical gas mixture for use in the imaging and/or treatment of tumors in patients inhaling the mixture, the mixture comprising from 20 to 70 vol.-% oxygen, from 1 - 8 vol.-% carbon dioxide, the balance being essentially helium.

US 6 001 332 A describes a medical gas mixture which comprises from 20 to 70 vol.-% oxygen, from 1 to 10 vol.-% carbon dioxide and the balance - except for incidental constituents not adversely affecting the basic properties of the gas mixture - being helium. This gas mixture is used in the treatment of asthma and for the enhancement of magnetic resonance imaging. Helium is preferably present in this gas mixture in an amount of at least 60 vol.-% in order to achieve optimum flow dynamics in the patient to be improved. The carbon dioxide content is preferably from about 3 vol.-% to 7 vol.-% and at least 3 vol.-% in case of using the composition in the therapy of asthma.

Macey et al.: "Hyperoxic brain effects Are Normalized by Addition of CO2", PloS Medicine, vol. 4, no. 5, 22 May 2007, pp. 828-835, XP55589703" describe the negative effect of the administration of pure (100%) oxygen. They propose the addition of carbon dioxide to therapeutic pure oxygen in order to overcome this effect disclose a gas mixture having an oxygen content of not more than 95% and a carbon dioxide content of not less than 5%.

It is also known that the determining factor for the control of respiratory activity under normal conditions is the arterial carbon dioxide partial pressure PaCO₂. For this reason a carbon dioxide-rich gas, such as the above-mentioned Carbogen, was supplied in the event of hyperventilation occurring during oxygen therapy in order to increase the PaCO₂ back to a normal value and thus, to stabilize the ventilation.

However, all these gas mixtures have a comparatively high carbon dioxide content of several percent in volume, suggesting that lower concentrations were not expected to have a measurable effect on the intended therapy. A problem inherent in such preparations are the pronounced physiological effects of the high carbon dioxide concentration, including inability to breath normally, high ventilation rates, panic and production of heat which may lead to the patient being unable or unwilling to continue with the treatment. This is particularly problematic for patients with obstructive pulmonary disease. At the same time, these gas mixtures have a significantly reduced oxygen content compared to pure oxygen. As a substitute for pure oxygen in oxygen therapy, they are therefore not or not easily suitable and they are not used in this way so far.

It is the aim of the present invention to provide a medical gas mixture for oxygen therapy that reduces the negative side effects observed with the pure oxygen administration. It is another aim of the present invention to provide a medical gas mixture which is more efficient in the delivery of oxygen to the tissue of patients than pure oxygen is, during the actual oxygen therapy.

These aims are reached by administration a gas mixture with a composition having the features of patent claim 1. Advantageous embodiments of the invention are claimed in the sub-claims.

The medical gas mixture according to the invention contains between 100 ppm and 2000 ppm (by volume) carbon dioxide, the remainder being essentially oxygen, and is used as an inhalation gas in the oxy-therapy for human use. The medical composition according to the invention therefore consists of two components, medical oxygen (*Oxigenium* Ph. Eur.) as an active pharmaceutical substance in an amount of at least 99.8 Vol.-% and medical carbon dioxide (*Carbonei dioxidum* Ph. Eur.) in the amount between 0.01 vol.-% and 0.2 vol.-% (100 - 2000 ppm) as an excipient or as a second active substance. Incidental constituents of further gaseous components in a total amount within the respective approval limits of, e.g. 300 ppm and/or within the respective detection limits may also be present in the gas mixture. It should be noted that in the scope of the present invention the gases specified as constituents of the inventive gas mixture are always to be understood as medical gases.

The invention is based on the surprising discovery that even a comparatively small addition of a maximum of 2000 ppm carbon dioxide in oxygen is sufficient to effectively counteract the negative side effects which arise during oxygen therapy. Without limiting the scope of the present invention, this finding is based on the assumption that an essential source of the side effects of hyperoxia seems to be the complete absence of the natural content of carbon dioxide in the medical oxygen currently administered to patients.

Thus, the solution according to the invention is to replace medical oxygen which is actually fully depleted of CO₂ with a pharmaceutical composition consisting mainly of oxygen, but which reflects the natural conditions and has a certain minimum content of carbon dioxide which is at least not far from the natural carbon dioxide content of air (currently, little above 400 ppmv CO₂ in air).

In contrast to the prevailing theories, the present invention furthermore assumes that the addition of a small amount of carbon dioxide significantly increases the efficacy of medical oxygen during oxygen therapy.

It can be demonstrated that at the origin of the deleterious effects of hyperoxia stands the fact that the natural content of carbon dioxide is completely missing in medical oxygen. Hyperventilation (and further physiological effects) is being induced by the lack of CO₂ in the inspired gas, as explained below.

When the respiration is switched from atmospheric air to 100% oxygen, a drop of 0.3 mmHg (40 Pa) is induced in the carbon dioxide partial pressure in the alveolar space, due to the fact that 0.04 vol.-% carbon dioxide contained in the atmospheric air is no longer contained in the inhaled gas. This drop affects the gradient between the carbon dioxide mean partial pressure in the lung capillaries and the alveolar partial pressure by increasing it. The increase can be calculated at around 5%. As the carbon dioxide partial pressure gradient is the driving force in the diffusion process, according to the first Fick's law, the increased gradient leads to an additional amount of CO₂ excreted from capillary in alveoli, resulting in an immediate higher alveolar partial pressure than normal. The respiratory control system adjusts the alveolar ventilation by increasing it up to the new level necessary to remove the extra-diffused (extra-excreted) CO₂, so that the arterial CO₂ pressure is to be corrected back to the initial PₐCO₂ setpoint value.

Since the supply of carbon dioxide into the blood vessels through metabolism remains essentially the same (about 200 ml/min for a healthy subject at rest) the patient exhales more CO₂ (a plus of 5%, i.e. 210 ml/min) during oxygen therapy than the value corresponding to the production by his metabolism. This discrepancy artificially induced by the CO₂-depletion in the respired gas corresponds in its effect to a 5%-hyperventilation. The patient will exhale more CO₂ than it would be necessary for his metabolism for the whole remaining duration of the oxygen therapy. This situation leads to a significant depletion of CO₂ of about 600 ml/hour, first in the blood (hypocapnia), then, due to CO₂ buffering, in the tissue, which can lead to vascular constriction, vascular resistance and finally to an impairment of oxygen supply. As the aim of oxygen therapy should be to increase the delivery of oxygen the overall result is a reduction of the therapeutic efficacy of oxygen administration.

In this context, it should be noted that the respiratory regulation system of mammals has been shaped, adapted and improved for carbon dioxide concentrations equal and higher than the present amount contained in the natural atmosphere, namely, at a value of at least 0.04 vol.-% CO₂ and never below this concentration.

By supplying the gas mixture according to the invention instead of pure oxygen during oxygen therapy, the mismatch between the carbon dioxide partial pressures in the pulmonary alveoli and the blood vessels is counteracted und thus, the negative side effects of the oxygen therapy mentioned above are minimized.

The positive effect of the carbon dioxide contained in the gas mixture according to the invention occurs from a portion of about 100 ppm CO₂ (0.01 vol.-%). A higher amount of CO₂ will stimulate the respiratory activity more and thus will increase the oxygen delivery. However, above an amount of ca. 2000 ppm CO₂ (0.2 vol.-%) the negative side effects of the increasing amount of carbon dioxide in the blood (hypercapnia) may outweigh the beneficial effect. Preferably, the carbon dioxide content is between 200 ppm (0,02 vol.-%) and 600 ppm (0,06 vol.-%), particular preferably the carbon dioxide content is in the range near the carbon dioxide content of the ambient atmosphere, i.e. between 300 ppm and 400 ppm (0.03 vol.-% and 0.04 vol.-%).

The oxygen content in the medical gas mixture according to the invention should be at least 99.8 vol.-%. An oxygen content of at least between 99.9 vol.-% and 99.95 vol.-%, in addition to the carbon dioxide content, will further reduce the amount of potentially harmful additional components. Thus, a preferred embodiment of the medical gas mixture according to the invention consists of 99.96 90,00 vol.-% oxygen and 0.04 vol.-% carbon dioxide.

The medical gas mixture according to the invention is used instead of, in addition to or in alternation with pure oxygen in all applications of medical oxygen therapy for human and in veterinary use. The use of the gas mixture according to the invention effectively counteracts various negative physiological effects of hyperoxia caused by the use of pure oxygen, in particular the well-known hyperoxia-induced hyperventilation, which in turn causes a number of additional negative side effects.

The medical gas mixture according to the invention is preferably used in a therapy of different types of acute and chronic hypoxia, in particular in anesthesiology, intensive care, treatment of cluster headache and/or for first aid therapy of diving accidents.

At use in such a therapy, the medical gas mixture according to the invention is preferably administrated at normobaric or hyperbaric conditions, using natural or mechanical ventilation, by using or not of specific medical devices for gas inhalation.

In particular, the medical gas mixture according to the invention is suited for use in a therapy of acute and chronic hypoxia, e.g. AMI (Acute Myocardial Infarction), cardiac arrest, stroke, ARDS (Acute Respiratory Distress Syndrome) and/or COPD (Chronic Obstructive Pulmonary Disease) or in the case of neonatal resuscitation. The gas mixture is also suitable as a substitute or supplement for pure oxygen for administration to patients in postoperative state, for critical ill patients or during the acute episodes of ARDS and/or COPD.

## Claims

1. Medical Gas mixture consisting of oxygen and carbon dioxide for use as an inhalation medicinal product (medicament), **characterized in that** the carbon dioxide content in oxygen is between 100 ppm and 2000 ppm.

2. The medical gas mixture for use according to Claim 1, **characterized in that** the carbon dioxide content is between 200 ppm and 600 ppm.

3. The medical gas mixture for use according to claim 1 or claim 2, **characterized in that** the carbon dioxide content is between 300 ppm and 400 ppm.

4. The medical gas mixture for use according to one of the previous claims, **characterized in that** the oxygen content is at least 99.8 vol.-%, preferably at least 99.95 vol.-%.

5. Medical gas mixture for use according to one of the preceding claims for use as an inhalation medicinal product (medicament) for the purpose of oxygen therapy.

6. Medical gas mixture for use in a therapy of acute and chronic hypoxia, in anesthesiology, intensive care, treatment of cluster headache and/or for first aid therapy of diving accidents,
**characterized in that** that the medical gas mixture comprises oxygen and carbon dioxide, where the oxygen content is at least 99.8 vol.-% and the carbon dioxide content is between 100 ppm and 2000 ppm.

7. Medical gas mixture for use according to claim 6, administered at normobaric or hyperbaric conditions, using natural or mechanical ventilation, by using or not of specific medical devices for gas inhalation.

## Patentansprüche

1. Medizinisches Gasgemisch, bestehend aus Sauerstoff und Kohlendioxid, zur Verwendung als Inhalationsarzneimittel (Medikament), **dadurch gekennzeichnet, dass** der Kohlendioxidgehalt in Sauerstoff zwischen 100 ppm und 2000 ppm liegt.

2. Medizinisches Gasgemisch zur Verwendung nach Anspruch 1, **dadurch gekennzeichnet, dass** der Kohlendioxidgehalt zwischen 200 ppm und 600 ppm liegt.

3. Medizinisches Gasgemisch zur Verwendung nach Anspruch 1 oder Anspruch 2, **dadurch gekennzeichnet, dass** der Kohlendioxidgehalt zwischen 300 ppm und 400 ppm liegt.

4. Medizinisches Gasgemisch zur Verwendung nach einem der vorherigen Ansprüche, **dadurch gekennzeichnet, dass** der Sauerstoffgehalt bei wenigstens 99,8 Vol.-%, bevorzugt wenigstens 99,95 Vol.-%, liegt.

5. Medizinisches Gasgemisch zur Verwendung nach einem der vorhergehenden Ansprüche zur Verwendung als Inhalationsarzneimittel (Medikament) zum Zwecke einer Sauerstofftherapie.

6. Medizinisches Gasgemisch zur Verwendung bei der Therapie von akuter und chronischer Hypoxie, in der Anästhesiologie, der Intensivmedizin, der Behandlung von Clusterkopfschmerzen und/oder als Erste-Hilfe-Therapie bei Tauchunfällen, **dadurch gekennzeichnet, dass** das medizinische Gasgemisch Sauerstoff und Kohlendioxid umfasst, wobei der Sauerstoffgehalt bei wenigstens 99,8 Vol.-% und der Kohlendioxidgehalt zwischen 100 ppm und 2000 ppm liegt.

7. Medizinisches Gasgemisch zur Verwendung nach Anspruch 6, verabreicht unter normobaren oder hyperbaren Bedingungen, wobei natürliche oder mechanische Beatmung verwendet wird, mit oder ohne Verwendung spezieller medizinischer Vorrichtungen zur Gasinhalation.

## Revendications

1. Mélange de gaz médical constitué d'oxygène et de dioxyde de carbone pour une utilisation en tant que produit (médicament) médicinal d'inhalation, **caractérisé en ce que** la teneur en dioxyde de carbone est comprise entre 100 ppm et 2 000 ppm.

2. Mélange de gaz médical pour une utilisation selon la revendication 1, **caractérisé en ce que** la teneur en dioxyde de carbone est comprise entre 200 ppm et 600 ppm.

3. Mélange de gaz médical pour une utilisation selon la revendication 1 ou la revendication 2, **caractérisé en ce que** la teneur en dioxyde de carbone est comprise entre 300 ppm et 400 ppm.

4. Mélange de gaz médical pour une utilisation selon l'une des revendications précédentes, **caractérisé en ce que** la teneur en oxygène est d'au moins 99,8 % en volume, préférablement d'au moins 99,95 % en volume.

5. Mélange de gaz médical pour une utilisation selon l'une des revendications précédentes pour une utilisation en tant que produit (médicament) médicinal d'inhalation à des fins de thérapie à l'oxygène.

6. Mélange de gaz médical pour une utilisation dans une thérapie d'une hypoxie aiguë et chronique, dans une anesthésiologie, des soins intensifs, le traitement de l'algie vasculaire de la face et/ou pour une thérapie de premier secours d'accidents de plongée, **caractérisé en ce que** le mélange de gaz médical comprend de l'oxygène et du dioxyde de carbone, où la teneur en oxygène est d'au moins 99,8 % en volume et la teneur en dioxyde de carbone est comprise entre 100 ppm et 2 000 ppm.

7. Mélange de gaz médical pour une utilisation selon la revendication 6, administré dans des conditions normobares ou hyperbares, en utilisant une ventilation naturelle ou mécanique, en utilisant ou non des dispositifs médicaux spécifiques pour l'inhalation de gaz.
